(19) Europäisches Patentamt
European Patent Office
Office européen des brevets

(11) EP 4 752 547 A1

(12) **EUROPEAN PATENT APPLICATION**

(43) Date of publication:
**03.06.2026 Bulletin 2026/23**

(21) Application number: **25218277.9**

(22) Date of filing: **25.11.2025**

(51) International Patent Classification (IPC):
***G01N 33/543*** *(2006.01)* ***G01N 33/545*** *(2006.01)*
***G01N 33/553*** *(2006.01)*

(52) Cooperative Patent Classification (CPC):
**G01N 33/545; G01N 33/54313; G01N 33/553**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **29.11.2024 JP 2024208569**

(71) Applicant: **Canon Kabushiki Kaisha**
**Tokyo, 146-8501 (JP)**

(72) Inventors:
- **NATORI, Ryo**
  **Tokyo (JP)**
- **YAMAUCHI, Fumio**
  **Tokyo (JP)**
- **KANAZAKI, Kengo**
  **Tokyo (JP)**
- **KASAI, Gota**
  **Tokyo (JP)**

(74) Representative: **WESER & Kollegen Patentanwälte PartmbB**
**Radeckestraße 43**
**81245 München (DE)**

(54) **PARTICLE FOR IMMUNOTURBIDIMETRY, REAGENT, TEST KIT, AND METHOD OF DETECTING TARGET SUBSTANCE**

(57) Provided are a particle for immunoturbidimetry, a reagent, a test kit, and a method of detecting a target substance that can each detect a trace component in a low-concentration region and improve storage stability. The particle for immunoturbidimetry is a particle for immunoturbidimetry including a first resin and titanium oxide, wherein the particle for immunoturbidimetry includes a first layer containing the first resin and a second layer containing the titanium oxide, wherein the second layer is arranged outside of the first layer, wherein the titanium oxide has a density of 3.40 g/cm$^3$ or less, and wherein a content of the titanium oxide in the particle for immunoturbidimetry is 10 mass% or more and 80 mass% or less.

EP 4 752 547 A1

## Description

TECHNICAL FIELD

The present disclosure relates to a particle for immunoturbidimetry, a reagent, a test kit, and a method of detecting a target substance.

BACKGROUND

In recent years, immunoturbidimetry has been drawing attention as a simple and rapid immunological testing method. As the immunoturbidimetry, there is used a method including mixing a dispersion of a particle having an antibody or an antigen as a ligand on its surface and a specimen that may contain a target substance (antigen or antibody). At this time, when the specimen contains the target substance (antibody or antigen), the particle causes an agglutination reaction, and hence the presence or absence of a disease can be identified by optically detecting the agglutination reaction as a variation in, for example, scattered light intensity, transmitted light intensity, or absorbance.
A polystyrene-based latex particle containing polystyrene as a main component has hitherto been used as a particle for immunoturbidimetry because the polystyrene-based latex particle is easy for sensitization (immobilization) of an antigen or an antibody and is relatively inexpensive, and its polymerization reaction is easy to control.
For example, there has been developed a particle using titanium oxide, which is well known as a high-refractive-index material. In Japanese Patent Laid-Open No. 2008-241357, there is a proposal of a particle for immunoturbidimetry in which a ligand is bound to a particle obtained by coating a carboxylic acid-modified polystyrene particle with a titanium oxide fine particle.

SUMMARY

In the immunoturbidimetry using the polystyrene-based latex particle, a problem has arisen in some cases in that a trace component in a low-concentration region cannot be detected. Accordingly, the development of a particle having sensitivity higher than that of the polystyrene-based latex particle has been demanded.
In order to solve the above-mentioned problem, it is important to increase a change in absorbance caused by particle aggregation along with the formation of an immune complex. In order to improve the change in absorbance, there has been developed a particle using a high-refractive index material.
In the method described in Japanese Patent Laid-Open No. 2008-241357, a particle containing a large amount of titanium oxide has a high density of titanium oxide and a large particle diameter in the first place. Accordingly, the sedimentation rate is fast when the particle is used as a test reagent, and there has been room for improvement in storage stability of the reagent.
The present disclosure has been made in view of the background art and the problems. Specifically, the present disclosure is directed to providing a particle for immunoturbidimetry, a reagent, a test kit, and a method of detecting a target substance that can each detect a trace component in a low-concentration region and improve storage stability in immunoturbidimetry.
The present disclosure in its first aspect provides a particle for immunoturbidimetry as specified in claim 1. Optional features are specified in claims 2 to 8.
The present disclosure in its second aspect provides a reagent as specified in claim 9.
The present disclosure in its third aspect provides a test kit as specified in claim 10.
The present disclosure in its fourth aspect provides a method of detecting a target substance in a specimen by in vitro diagnosis as specified in claims 11 and 12.
According to the present disclosure, there can be provided a particle for immunoturbidimetry, a reagent, a test kit, and a method of detecting a target substance that can each detect a trace component in a low-concentration region of a target substance and improve storage stability.
Features of the present disclosure will become apparent from the following description of embodiments. The following description of embodiments is described by way of example.

DESCRIPTION OF THE EMBODIMENTS

Embodiments of the present disclosure are described in detail below. However, the technical scope of the present disclosure is not limited to the embodiments.
A particle for immunoturbidimetry according to the present disclosure is a particle for immunoturbidimetry including a first resin and titanium oxide, wherein the particle for immunoturbidimetry includes a first layer containing the first resin and a second layer containing the titanium oxide, wherein the second layer is arranged outside of the first layer,

wherein the titanium oxide has a density of 3.40 g/cm$^3$ or less, and wherein a content of the titanium oxide in the particle for immunoturbidimetry is 10 mass% or more and 80 mass% or less.

The inventors have found that when the particle includes the first resin as its core, that is, the first layer, and includes a layer containing titanium oxide having a density of 3.40 g/cm$^3$ or less as the second layer formed outside thereof, a trace component in a low-concentration region of a target substance can be detected and storage stability can be improved. The reason is conceived to be as described below. The arrangement of a resin layer as the first layer and a layer containing titanium oxide having a density of 3.40 g/cm$^3$ or less as the second layer can achieve a system in which a change in turbidity at the time of particle aggregation is large, though the particle density is smaller than that in the case of a particle including only titanium oxide. It is conceived that this system has enabled the detection of a trace component in a low-concentration region of a target substance and the improvement in storage stability.

The titanium oxide in the particle for immunoturbidimetry of the present disclosure has a density of 3.40 g/cm$^3$ or less. In general, titanium oxide circulating on the market is produced by, for example, a sulfate method or a chlorine method, and has a crystal structure, such as an anatase type, a brookite type, or a rutile type, with a high crystallinity. Titanium oxide having a high crystallinity has a high density and is different from the titanium oxide to be used in the present disclosure. The inventors have conceived that the incorporation of low-density, that is, lightweight titanium oxide in a certain range enables the detection of a trace component in a low-concentration region of a target substance and the improvement in storage stability, and have arrived at the present disclosure.

The density of the titanium oxide of the present disclosure is preferably 1.50 g/cm$^3$ or more and 3.40 g/cm$^3$ or less, more preferably 2.50 g/cm$^3$ or more and 3.40 g/cm$^3$ or less in order that a trace component in a low-concentration region of a target substance can be detected.

The titanium oxide to be used in the present disclosure is not particularly limited as long as its density falls within the above-mentioned ranges, but may be obtained by, for example, a method of hydrolyzing a metal oxide precursor containing titanium, which is called a sol-gel method. That is, the titanium oxide to be used in the present disclosure may be obtained by allowing a metal oxide precursor, an oxygen-containing organic solvent, and water to coexist and performing a hydrolysis reaction.

Examples of the metal oxide precursor include a metal chloride, a metal acetate, a metal alkoxide, and a metal hydroxide. Of those, a metal alkoxide, a metal acetate, and a metal hydroxide are preferably used from the viewpoint of impurities (e.g., a chloride) that are by-produced. Of those, a metal alkoxide represented by the chemical formula $M_x(OR)_y$ (where M represents a metal element, R represents an alkyl group, and "x" and "y" each independently represent an integer of 1 or more and 4 or less), a metal hydroxide represented by the chemical formula $M_{x'}(OH)_{y'}\cdot nH_2O$ (where M represents a metal element, x' and y' each independently represent an integer of 1 or more and 4 or less, and "n" represents an integer of 1 or more), and a compound containing the above-mentioned metal alkoxide and/or metal hydroxide are particularly preferred. Specific examples thereof include titanium methoxide, titanium ethoxide, titanium diisopropoxide bis(2,4-pentanedionate), titanium diisopropoxide bis(ethyl acetoacetate), titanium-n-butoxide, titanium isopropoxide, titanium methoxypropoxide, titanium-n-nonyloxide, titanium-n-propoxide, titanium stearyloxide, titanium triisostearyl isopropoxide, and titanium trimethylsiloxide.

Examples of the oxygen-containing organic solvent include alcohols, ketones, aldehydes, ethers, esters, and siloxanes.

The particle for immunoturbidimetry according to the present disclosure includes a first layer containing a first resin and a second layer containing titanium oxide, and the second layer is arranged outside of the first layer. When the particle includes the first resin as its core, that is, the first layer, and includes titanium oxide as the second layer arranged outside thereof, sufficient amounts of the resin and titanium oxide necessary for detecting a trace component in a low-concentration region and improving storage stability can be incorporated.

The particle for immunoturbidimetry according to the present disclosure is not particularly limited as long as the particle includes the second layer outside of the first layer, but may be obtained by, for example, allowing the first resin to coexist in the hydrolysis reaction described above. That is, the particle may be obtained by allowing the metal oxide precursor, the oxygen-containing organic solvent, water, and the first resin to coexist and performing the hydrolysis reaction. The first resin may be obtained by a general polymer particle production method, such as an emulsion polymerization method, a soap-free polymerization method, a dispersion polymerization method, a suspension polymerization method, a phase inversion emulsification method, a wet pulverization method, or a dry pulverization method, and is not particularly limited. The first resin obtained by each of the emulsion polymerization method and the soap-free polymerization method is preferred because a volume average particle diameter desired for a particle for immunoturbidimetry is obtained and a uniform particle size distribution is obtained.

The range of the volume average particle diameter preferred for the particle for immunoturbidimetry is 150 nm or more and 1,000 nm or less, more preferably 180 nm or more and 800 nm or less. In addition, the range of the particle size distribution preferred for the particle for immunoturbidimetry is a value of the ratio "volume average particle diameter/-number average particle diameter" of 1.00 or more and 1.25 or less.

The first resin of the present disclosure is not particularly limited, but preferably has a high refractive index.

Preferred specific structures for that purpose may include a styrene structure, a fluorene structure, a dinaphthothiophene structure, a naphthalene structure, an anthracene structure, and a phenanthrene structure.

The content of the titanium oxide in the particle for immunoturbidimetry of the present disclosure is 10 mass% or more and 80 mass% or less, preferably 21 mass% or more and 60 mass% or less. When the content of the titanium oxide is less than 10 mass%, it may be difficult to detect a trace component in a low-concentration region of a target substance. When the content of the titanium oxide is more than 80 mass%, the sedimentation rate may increase to cause a problem with storage stability.

The density of the particle for immunoturbidimetry according to the present disclosure is preferably 1.20 g/cm$^3$ or more and 1.54 g/cm$^3$ or less.

With regard to the titanium oxide of the present disclosure, when a sufficient amount of the titanium oxide having a density of 3.40 g/cm$^3$ or less is incorporated into the particle and the particle density is set within the above-mentioned range, both of an improvement in sensitivity and the suppression of sedimentation can be achieved.

The circularity of the particle for immunoturbidimetry according to the present disclosure is preferably 0.85 or more and 1.00 or less. When the circularity of the particle is set within the above-mentioned range, the particles smoothly approach each other accompanying an antigen-antibody reaction, and hence a higher sensitivity can be achieved.

In addition, the particle for immunoturbidimetry according to the present disclosure preferably includes a third layer containing a second resin outside of the second layer containing titanium oxide. When the particle includes the third layer, the adsorption rate of an antibody or an antigen serving as a biosensor is improved, and a higher sensitivity can be achieved.

The second resin in the third layer preferably has a structure represented by the following formula (1). This can promote a further improvement in adsorption rate of an antibody or an antigen serving as a biosensor.

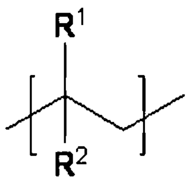

(1)

In the formula (1), $R^1$ represents a hydrogen atom or a methyl group, and $R^2$ represents a structure having a phenyl group that may be substituted or a structure having an ester group.

In addition, the second resin in the third layer more preferably has at least one of a structure represented by the following formula (1-1) and a structure represented by the following formula (1-2) as the structure represented by the formula (1):

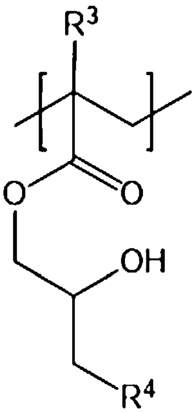

(1-1)

in the formula (1-1), $R^3$ represents a hydrogen atom or a methyl group, and $R^4$ represents a structure having a hydroxy group or a structure having a carboxy group; and

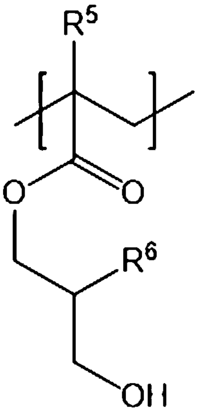

(1-2)

in the formula (1-2), $R^5$ represents a hydrogen atom or a methyl group, and $R^6$ represents a structure having a hydroxy group or a structure having a carboxy group.

When the coating resin has at least one of the structure represented by the formula (1-1) and the structure represented by the formula (1-2), the coating resin satisfactorily interacts with the titanium oxide, and can uniformly coat the titanium oxide without exposure thereof. This can suppress the desorption of the resin. Specific examples of the structure represented by the formula (1-1) or (1-2) are shown in the following formulae (1-A-1) to (1-A-12), but the present disclosure is not limited thereto.

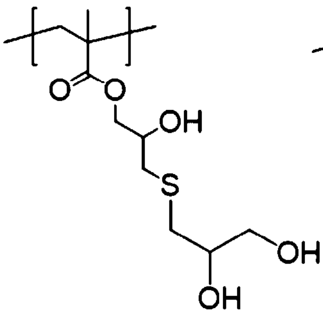

(1-A-1)

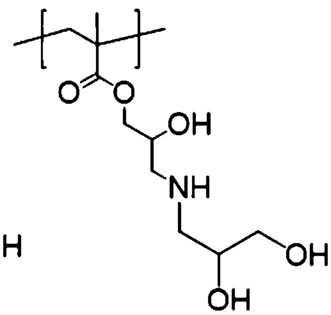

(1-A-2)

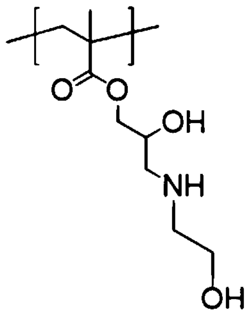

(1-A-3)

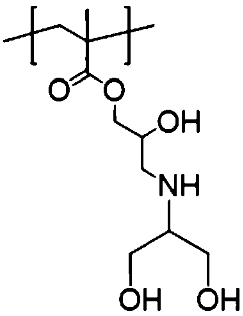

(1-A-4)

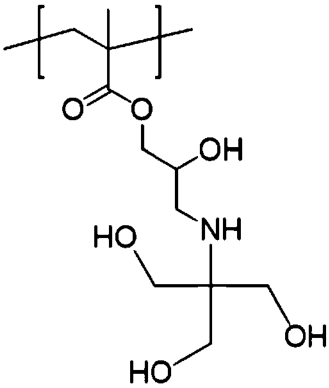

(1-A-5)

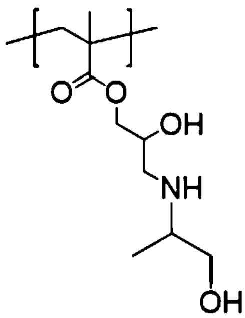

(1-A-6)

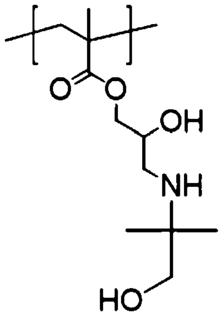

(1-A-7)

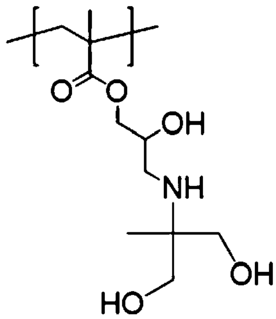

(1-A-8)

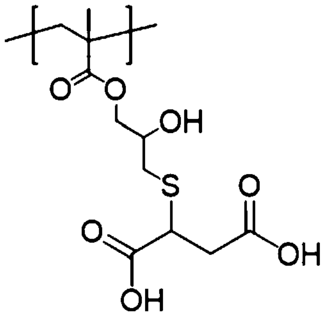

(1-A-9)

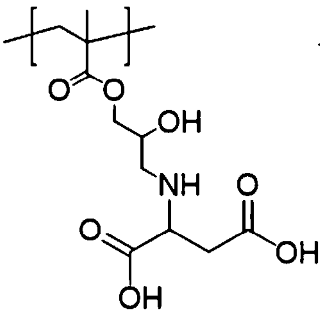

(1-A-10)

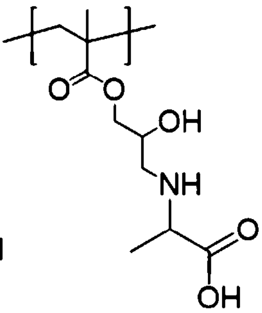

(1-A-11)

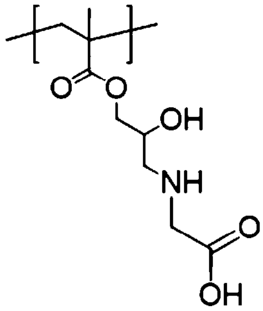

(1-A-12)

Further, the second resin preferably has at least one of a structure represented by the following formula (2) and a structure represented by the following formula (3).

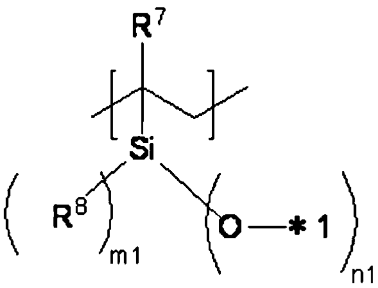

(2)

In the formula (2), $R^7$ represents a hydrogen atom or a methyl group, n1 represents an integer of 1 or more and 3 or less, m1 represents an integer of 0 or more and 2 or less, n1+m1 is 3, *1s each independently represent a bond to a titanium atom or a silicon atom, or represent a hydrogen atom, a methyl group, or an ethyl group, and $R^8$s each independently represent a methyl group or an ethyl group. That is, the structure represented by the formula (2) may be bonded to a titanium atom of titanium oxide via an oxygen atom, or may be bonded to a silicon atom of another structure represented by the formula (2) or the structure represented by the formula (3) via an oxygen atom.

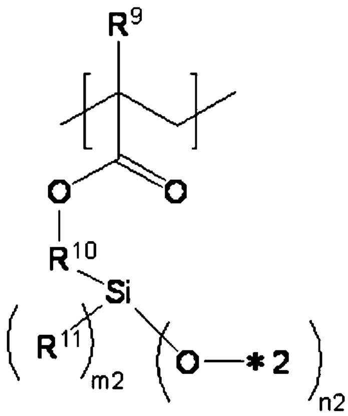

(3)

In the formula (3), $R^9$ represents a hydrogen atom or a methyl group, $R^{10}$ represents a single bond, a phenylene group, or an alkylene group having 3 or less carbon atoms, n2 represents an integer of 1 or more and 3 or less, m2 represents an integer of 0 or more and 2 or less, n2+m2 is 3, *2s each independently represent a bond to a titanium atom or a silicon atom, or represent a hydrogen atom, a methyl group, or an ethyl group, and $R^{11}$s each independently represent a methyl group or an ethyl group. That is, the structure represented by the formula (3) may be bonded to a titanium atom of titanium oxide via an oxygen atom, or may be bonded to a silicon atom of another structure represented by the formula (3) or the structure represented by the formula (2) via an oxygen atom.

The resin further containing the structure represented by the formula (2) or the structure represented by the formula (3) in addition to the structure represented by the formula (1-1) or the structure represented by the formula (1-2) is a vinyl-based polymer and has an alkoxysilane. The exposure of the titanium oxide is further suppressed by the interaction with the titanium oxide, and uniform coating is achieved.

The particle having the structure represented by the formula (1-1) or the structure represented by the formula (1-2) may be obtained by, for example, causing a titanium oxide-containing particle and a monomer to coexist in an aqueous medium and adding a polymerization initiator thereto to perform a polymerization reaction, that is, by performing seed polymerization. The monomer is not particularly limited, but for example, the particle may be obtained by performing seed polymerization using glycidyl (meth)acrylate as the monomer and then performing the ring-opening reaction of a glycidyl group. In addition, the monomers may be used alone or as a mixture thereof. The monomer to be mixed is not particularly limited, but when the monomer contains the structure represented by the formula (2) or the structure represented by the formula (3), the particle is obtained by mixing monomers from which the structure represented by the formula (2) or the structure represented by the formula (3) is derived and performing seed polymerization by copolymerization of two or more kinds of monomers. The monomer from which the structure represented by the formula (2) or the structure represented by the formula (3) is derived is not particularly limited as long as the monomer has a structure from which the structure represented by the formula (2) or the structure represented by the formula (3) is derived. Examples thereof include vinyltrimethoxysilane, vinyltriethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, and 3-acryloxypropyltrimethoxysilane. Those monomers may also be used alone or in combination thereof.

A particle including the second resin may also be obtained by, for example, allowing the titanium oxide-containing particle and the second resin to coexist in an aqueous medium to perform an adsorption reaction therebetween. In this case, the second resin is not particularly limited as long as the second resin is soluble in an aqueous medium and can be adsorbed to the second layer containing titanium oxide. For example, a water-soluble polymer such as poly(sodium acrylate), a polysaccharide such as sodium carboxymethyldextran, and a protein such as bovine serum albumin may be used.

The particle for immunoturbidimetry according to the present disclosure may be a pre-sensitization particle to which no ligand is imparted, or may be an affinity particle further including a ligand on its surface. The affinity particle has selectively or specifically high affinity to a target substance by virtue of the ligand added to the surface of the particle. In particular, it is preferred that the ligand be added to the surface of the particle by a chemical bond.

The ligand in the present disclosure is a compound that specifically binds to a receptor of a specific target substance. A site where the ligand binds to the target substance is fixed, and has selectively or specifically high affinity. Examples thereof include an antigen and an antibody, an enzyme protein and a substrate therefor, a signal substance, such as a hormone or a neurotransmitter, and a receptor therefor, and a nucleic acid, but the ligand in the present disclosure is not limited thereto. An example of the nucleic acid is deoxyribonucleic acid. The affinity particle in the present disclosure has selectively or specifically high affinity to the target substance. It is preferred that the ligand in the present disclosure be any one of an antibody, an antigen, and a nucleic acid.

In addition, a reagent according to the present disclosure is a reagent in which the particle for immunoturbidimetry

according to the present disclosure is dispersed in an aqueous solution.

The test object of the reagent is not particularly limited. An example of the reagent is an in vitro diagnostic drug, and examples thereof include an antigen detection reagent using an antigen as a test object and an antibody detection reagent using an antibody as a test object. The reagent may be a reagent in which a pre-sensitization particle free of a ligand is dispersed in an aqueous solution, assuming that a user adds a desired ligand such as an antibody to the particle. Alternatively, assuming a specific target substance, the reagent may be such a reagent that an affinity particle, in which a ligand has been added, is dispersed in an aqueous solution in advance.

The reagent according to the present disclosure includes the particle for immunoturbidimetry (pre-sensitization particle or affinity particle) according to the present disclosure and a dispersion medium for dispersing the particle for immunoturbidimetry. The reagent according to the present disclosure may include a third substance, such as a solvent or a blocking agent, in addition to the particle for immunoturbidimetry according to the present disclosure to the extent that the object of the present disclosure can be achieved. Two or more kinds of third substances, such as a solvent and a blocking agent, may be incorporated in combination. Examples of the dispersion medium to be used include various buffers, such as a phosphate buffer, a glycine buffer, a Good's buffer, a Tris buffer, and an ammonia buffer, but the dispersion medium in the reagent is not limited thereto.

A test kit according to the present disclosure includes the reagent according to the present disclosure and a container that accommodates the reagent. In addition to the reagent (hereinafter referred to as "reagent 1") described above, the test kit may include a reaction buffer (hereinafter referred to as "reagent 2"). A sensitizer may be incorporated into each of both the reagent 1 and the reagent 2, or any one of the reagents. In addition, the test kit in the present disclosure may include a positive control, a negative control, a serum diluent, a primary antibody, a secondary antibody, and the like in addition to the reagent 1 and the reagent 2. As a medium for the positive control or the negative control, a solvent may be used in addition to serum and physiological saline each free of a target substance that may be measured.

A method of detecting a target substance according to the present disclosure may be a method of detecting a target substance in a specimen by in vitro diagnosis, the method including mixing the reagent according to the present disclosure and a specimen that may contain the target substance. In addition, the method of detecting a target substance according to the present disclosure may be a method of detecting a target substance in a specimen by in vitro diagnosis, the method including: mixing the reagent according to the present disclosure and a specimen that may contain the target substance to provide a mixed solution; irradiating the mixed solution with light; and detecting at least one of transmitted light and scattered light from the light with which the mixed solution has been irradiated.

(Method of measuring Content of Titanium Oxide in Particle for Immunoturbidimetry)

An example of a method of measuring the content of the titanium oxide in the particle for immunoturbidimetry according to the present disclosure is described. The content of the titanium oxide in the particle is measured with a thermogravimetric analyzer. For example, STA200 (manufactured by Hitachi High-Tech Science Corporation) is used. 5 Milligrams to 10 mg of a cured product is weighed in an aluminum pan and measured in a nitrogen atmosphere. The temperature conditions are to hold the temperature at 30°C for 30 minutes, then increase the temperature from 30°C to 500°C at 10°C/min, and subsequently hold the temperature at 500°C for 10 minutes.

As the evaluation, the content of the titanium oxide in the particle may be measured from the mass loss of a resin component at 300°C.

(Method of measuring Density of Titanium Oxide in Particle for Immunoturbidimetry)

An example of a method of measuring the density of the titanium oxide in the present disclosure is described. The density of the titanium oxide may be measured by separating the titanium oxide from the particle for immunoturbidimetry and performing dry density measurement on the separated titanium oxide by a constant volume expansion method. For example, AccuPyc II (manufactured by Shimadzu Corporation) is used and measurement is performed at 25°C with an average of 10 measurements.

A method of separating the titanium oxide from the particle is not particularly limited, but the titanium oxide in the particle may be taken out by, for example, dissolving and washing the resin in the particle with a solvent.

In addition, the density of the titanium oxide may be calculated by the following formula.

$$D_P=W_T\times D_T+(1-W_T)\times D_R$$

$D_P$: density of the particle measured by the method given herein as an example
$D_T$: titanium oxide density
$D_R$: resin density

$W_T$: content of the titanium oxide in the particle measured by the method given herein as an example

The resin density is calculated as 1.10 g/cm$^3$.

(Method of measuring Density of Particle for Immunoturbidimetry)

An example of a method of measuring the density of the particle for immunoturbidimetry in the present disclosure is described. The density of the particle is measured by, for example, dry density measurement by a constant volume expansion method. For example, AccuPyc II (manufactured by Shimadzu Corporation) is used and measurement is performed at 25°C with an average of 10 measurements.

(Methods of measuring Volume Average Particle Diameter and Particle Size Distribution of Particles for Immunoturbidimetry in Aqueous Dispersion)

A method of measuring the volume average particle diameter (Dv) of the particles for immunoturbidimetry in the present disclosure is described. The Dv of particles present in an aqueous dispersion is measured by a dynamic light scattering method. For example, Zetasizer (Zetasizer ultra: manufactured by Malvern Panalytical Ltd.) is used and measurement is performed at 25°C.

In addition, the particle size distribution of the particles in the present disclosure is calculated by measuring a number average particle diameter (Dn) by the above-mentioned dynamic light scattering method to determine the ratio of Dv to Dn (Dv/Dn).

(Method of measuring Circularity of Particle for Immunoturbidimetry)

A method of measuring the circularity of the particle for immunoturbidimetry in the present disclosure is described. 500 Particles are extracted from a scanning electron microscope (SEM) image, the circularity of each particle is calculated through use of image analysis software, and an average value of the 500 particles is adopted as the circularity. The circularity is calculated through use of, for example, S4800 manufactured by Hitachi High-Tech Corporation as a scanning electron microscope, and for example, Image-J as image analysis software.

[Examples]

The present disclosure is described in detail below by way of Examples, but the present disclosure is not limited to these Examples.

[Particle Preparation Example]

(Preparation of Particle 1)

(Step of forming First Layer containing Resin)

12.68 Grams of styrene (St: Kishida Chemical Co., Ltd.), 0.23 g of divinylbenzene (DVB: Kishida Chemical Co., Ltd.), and 1,512.02 g of ion-exchanged water were weighed into a 2 L four-necked separable flask to prepare a mixed solution. Oxygen was removed from the inside of the four-necked separable flask by holding the mixed solution at 70°C under stirring at 140 rpm and performing a nitrogen flow at a flow rate of 200 ml/min. Next, a dissolved solution of 0.55 g of V-50 (FUJIFILM Wako Pure Chemical Corporation) dissolved in 20 g of ion-exchanged water, which had been separately prepared, was added to the mixed solution to start soap-free polymerization. A dispersion of a first layer-forming particle 1 containing a copolymer of styrene and divinylbenzene was obtained by a reaction for 23 hours from the start of the polymerization. Part of the dispersion was collected and evaluated by using dynamic light scattering (Zetasizer ultra: Malvern Panalytical Ltd.), and as a result, the volume average particle diameter was 190 nm.

(Step of forming Second Layer containing Titanium Oxide)

The first layer-forming particle 1 was adjusted with ion-exchanged water to have a solid content concentration of 0.6%. 20 Grams of the dispersion was mixed into 404.50 g of ethanol (Kishida Chemical Co., Ltd.) containing 0.2% of polyvinylpyrrolidone K-30 (PVP K-30: Kishida Chemical Co., Ltd.), and the mixture was held at 70°C while being stirred at 140 rpm. Next, a solution of 5.0 ml of titanium(IV) n-butoxide, monomer (TBOT: Kishida Chemical Co., Ltd.) mixed into 197.50 g of ethanol, which had been separately prepared, was added to the mixed solution to start a sol-gel reaction. The

reaction was performed for 24 hours from the start of the sol-gel reaction, and the particle dispersed in the mixed solution was separated with a centrifuge and re-dispersed in ethanol. Further, the operation of separating the particle from the dispersion with a centrifuge and re-dispersing the particle in ion-exchanged water was repeated twice for purification to provide a second layer-forming particle 1. The second layer-forming particle 1 was stored in a state of an aqueous dispersion finally adjusted to 5.0 mass%. Part of the dispersion was collected and the dynamic scattering of the second layer-forming particle 1 was evaluated, and as a result, the volume average particle diameter was 200 nm. In addition, when the metal oxide content was evaluated by using differential thermal-thermogravimetric simultaneous analysis (NEXTA STA200RV: Hitachi High-Tech Corporation), the content was 45% of the particle weight.

(Step of forming Third Layer containing Resin)

Adjustment was performed with ion-exchanged water to provide 149.55 g of the second layer-forming particle 1 dispersion having a solid content concentration of 0.2%. 0.135 Gram of glycidyl methacrylate (GMA: Kishida Chemical Co., Ltd.) and 0.015 g of 3-methacryloxypropyltrimethoxysilane (MPS: Shin-Etsu Chemical Co., Ltd.) were added thereto, and the mixture was held at 70°C while being stirred at 100 rpm. Oxygen was removed from the inside of the four-necked separable flask by performing a nitrogen flow at a flow rate of 200 ml/min. Then, a dissolved solution of 0.03 g of V-50 dissolved in 0.3 g of ion-exchanged water, which had been separately prepared, was added to the mixed solution to start shell formation. A dispersion containing a third layer-forming particle 1 was obtained by continuing stirring for 18 hours after the start of the reaction.

(Step of imparting Reactive Functional Group)

An aqueous solution in which mercaptosuccinic acid (MSA: Wako Pure Chemical Industries, Ltd.) was dissolved (the total number of moles of MSA was equal to the number of moles of the glycidyl methacrylate), which had been prepared in advance, was added to the dispersion containing the third layer-forming particle 1. Triethylamine (Kishida Chemical Co., Ltd.) was added thereto to adjust the pH to 10. Next, the mixture was increased in temperature to 70°C while being stirred at 800 rpm. The mixture was further kept in this state for 18 hours to provide a dispersion containing a particle 1.

(Step of washing Particle)

The operation of separating the particle 1 from the dispersion with a centrifuge and re-dispersing the particle 1 in ion-exchanged water was repeated eight times, and the particle concentration was finally adjusted to 5.0 mass%. Thus, a dispersion of the particle 1 was obtained.

(Preparation of Particle 2 to Particle 6)

In the step of forming a third layer containing a resin, 0.15 g of glycidyl methacrylate was added instead of the addition of 0.135 g of glycidyl methacrylate and 0.015 g of 3-methacryloxypropyltrimethoxysilane. A dispersion of a particle 2 was obtained by the same operation as that for the particle 1 except for the foregoing.

In the step of forming a third layer containing a resin, 0.135 g of styrene and 0.015 g of methacrylic acid (MAA: Kishida Chemical Co., Ltd.) were added instead of the addition of 0.135 g of glycidyl methacrylate and 0.015 g of 3-methacryloxypropyltrimethoxysilane. In addition, the step of imparting a reactive functional group was not performed. A dispersion of a particle 3 was obtained by the same operation as that for the particle 1 except for the foregoing.

A dispersion of a particle 4 was obtained by the same operation as that for the particle 2 except that the amount of titanium (IV) n-butoxide, monomer was changed from 5.0 ml to 3.0 ml in the step of forming a second layer containing titanium oxide.

A dispersion of a particle 5 was obtained by the same operation as that for the particle 2 except that the amount of titanium (IV) n-butoxide, monomer was changed from 5.0 ml to 7.5 ml in the step of forming a second layer containing titanium oxide.

A dispersion of a particle 6 was obtained by the same operation as that for the particle 2 except that the amount of titanium (IV) n-butoxide, monomer was changed from 5.0 ml to 2.0 ml in the step of forming a second layer containing titanium oxide.

(Preparation of Particle 7)

Bovine serum albumin (hereinafter sometimes abbreviated as "BSA") was used for a third layer containing a resin. First, 1 mL of ion-exchanged water was added to 30 mg of BSA to prepare a BSA aqueous solution. 0.5 Milliliter of the

BSA aqueous solution was loaded into a 1.5 mL tube, and then 0.5 mL of a 2.0 mass% solution of the second layer-forming particle 1 was added thereto. After thorough mixing, the dispersion was left to stand overnight at room temperature. Next, the operation of separating the particle from the dispersion with a centrifuge and re-dispersing the particle in ion-exchanged water was repeated three times, and the particle concentration was finally adjusted to 5.0 mass%. Thus, a dispersion of a particle 7 including a third layer containing BSA was obtained.

(Preparation of Particle 8)

Poly(sodium acrylate) (hereinafter sometimes abbreviated as "PAANa") was used for a third layer containing a resin. The molecular weight of PAANa was from 22,000 to 66,000. First, 10 mL of ion-exchanged water was added to 10 mg of PAANa to prepare a PAANa aqueous solution. 0.5 Milliliter of the PAANa aqueous solution was loaded into a 1.5 mL tube, and then 0.5 mL of a 2.0 mass% solution of the second layer-forming particle 1 was added thereto. After thorough mixing, the dispersion was left to stand overnight at room temperature. Next, the operation of separating the particle from the dispersion with a centrifuge and re-dispersing the particle in ion-exchanged water was repeated three times, and the particle concentration was finally adjusted to 5.0 mass%. Thus, a dispersion of a particle 8 including a third layer containing PAANa was obtained.

(Preparation of Particle 9)

Sodium carboxymethyldextran (hereinafter sometimes abbreviated as "Dex") was used for a third layer containing a resin. The molecular weight of Dex was 40,000. First, 1 mL of ion-exchanged water was added to 20 mg of Dex to prepare a Dex aqueous solution. 0.5 Milliliter of the Dex aqueous solution was loaded into a 1.5 mL tube, and then 0.5 mL of a 2.0 mass% solution of the second layer-forming particle 1 was added thereto. After thorough mixing, the dispersion was left to stand overnight at room temperature. Next, the operation of separating the particle from the dispersion with a centrifuge and re-dispersing the particle in ion-exchanged water was repeated three times, and the particle concentration was finally adjusted to 5.0 mass%. Thus, a dispersion of a particle 9 including a third layer containing Dex was obtained.

(Preparation of Particle 10 and Particle 11)

A dispersion of a particle 10 was obtained by the same operation as that for the particle 2 except that the amount of styrene was changed from 12.68 g to 101.44 g and the amount of divinylbenzene was changed from 0.23 g to 1.84 g in the step of forming a first layer containing a resin.

A dispersion of a particle 11 was obtained by the same operation as that for the particle 2 except that divinylbenzene was not added and styrene was changed to methyl methacrylate (MMA: Kishida Chemical Co., Ltd.) in the step of forming a first layer containing a resin.

[Comparative Particle Preparation Examples]

50 Microliters of a titanium oxide fine particle dispersion and 950 μL of a γ-aminopropyltriethoxysilane aqueous solution (pure water containing 1 mass% of γ-aminopropyltriethoxysilane) were loaded into a centrifuge tube, and the mixture was stirred at 100 rpm for 1 hour through use of a tube roller at room temperature (20°C to 30°C). Next, the centrifuge tube was centrifuged at 15,000 rpm (about 20,000 G) for 15 minutes, and then the supernatant was removed by aspiration. 1,500 Microliters of an acetate buffer solution (0.01 M, pH: 5.0) was added to the precipitate, which was re-dispersed through use of a vortex mixer, and centrifuged again at 15,000 rpm (about 20,000 G) for 15 minutes, and the supernatant was removed by aspiration. Further, this step (the step of adding the acetate buffer solution, centrifuging, and removing the supernatant) was repeated three times. 500 Microliters of a phosphate buffer solution (0.01 M, pH: 6.0) was added to the obtained precipitate, which was dispersed for 2 minutes with an ultrasonic crusher and re-dispersed to provide an amino group-introduced titanium oxide fine particle dispersion.

The following materials were added to a centrifuge tube, and the mixture was stirred at 100 rpm for 1 hour through use of a tube roller at room temperature (20°C to 30°C).

| | |
|---|---|
| ·Carboxy-modified polystyrene fine particle dispersion [product name: manufactured by JSR Corporation, carboxy-modified latex, volume average of contained fine particles: 200 nm, fine particle content: 10%] (B-1)"IMMUTEX",particle diameter | 100 μL |
| ·HEPES buffer solution containing 1% bovine serum albumin | 900 μL |
| ·Soluble carbodiimide solution [1 M aqueous solution-dimethylaminopropyl)carbodiimide hydrochloride; the same applies hereinafter]of 1-ethyl-3-(3- | 500 μL |

Next, the centrifuge tube was centrifuged at 15,000 rpm (about 20,000 G) for 15 minutes, and then the supernatant was removed by aspiration. 1,500 Microliters of pure water was added to the precipitate, which was re-dispersed through use of a vortex mixer. The resultant was centrifuged again at 15,000 rpm (about 20,000 G) for 15 minutes, the supernatant was removed by aspiration, and 500 μL of a HEPES buffer solution containing 1% bovine serum albumin was added to this precipitate. The resultant was dispersed for 2 minutes with an ultrasonic crusher and re-dispersed to provide a carboxy group-activated polystyrene fine particle dispersion.

88 Microliters of the amino group-introduced titanium oxide fine particle dispersion and 500 μL of the carboxy group-activated polystyrene fine particle dispersion were loaded into a centrifuge tube, and the mixture was stirred at 100 rpm for 2 hours through use of a tube roller at room temperature (20°C to 30°C). Next, the centrifuge tube was centrifuged at 15,000 rpm (about 20,000 G) for 15 minutes, and then the supernatant was removed by aspiration. 1,500 Microliters of a phosphate buffer solution (0.1 M, pH: 7.1) was added to the precipitate, which was re-dispersed through use of a vortex mixer to provide a titanium oxide-polystyrene composite fine particle dispersion.

The titanium oxide-polystyrene composite fine particle dispersion was centrifuged by a density gradient centrifugation method, and a fraction having a density of 1.60 (content of titanium oxide in the composite particle: 20%) was obtained as a comparative particle 1. In the same manner, a fraction having a density of 1.20 (content of titanium oxide in the composite particle: 5%) was obtained as a comparative particle 2.

The physical properties of the obtained particles 1 to 11 and comparative particles 1 and 2 are collectively shown in Tables 1-1 and 1-2.

Table 1-1

| | Particle Preparation Example | | | | | | |
|---|---|---|---|---|---|---|---|
| | Particle 1 | Particle 2 | Particle 3 | Particle 4 | Particle 5 | Particle 6 | Particle 7 |
| First layer resin | PSt | PSt | PSt | PSt | PSt | PSt | PSt |
| Third layer resin | GMA MPS | GMA | PSt MAA | GMA | GMA | GMA | BSA |
| Density of titanium oxide ($g/cm^3$) | 2.77 | 2.76 | 2.76 | 2.75 | 2.74 | 2.76 | 2.72 |
| Content of titanium oxide (mass%) | 32 | 32 | 32 | 21 | 44 | 12 | 34 |
| Particle density ($g/cm^3$) | 1.62 | 1.62 | 1.62 | 1.42 | 1.82 | 1.28 | 1.62 |
| Volume average particle diameter (nm) | 210 | 210 | 210 | 200 | 220 | 200 | 210 |
| Circularity | > 0.85 | > 0.85 | > 0.85 | > 0.85 | > 0.85 | > 0.85 | > 0.85 |

Table 1-2

| | Particle Preparation Example | | | | Comparative Particle Preparation Example | |
|---|---|---|---|---|---|---|
| | Particle 8 | Particle 9 | Particle 10 | Particle 11 | Comparative particle 1 | Comparative particle 2 |
| First layer resin | PSt | PSt | PSt | MMA | - | - |
| Third layer resin | PAANa | Dex | GMA | GMA | - | - |
| Density of titanium oxide ($q/cm^3$) | 2.72 | 2.72 | 2.76 | 2.77 | 3.68 | 3.48 |
| Content of titanium oxide (mass%) | 31 | 33 | 18 | 21 | 20 | 5 |
| Particle density ($g/cm^3$) | 1.62 | 1.62 | 1.39 | 1.41 | 1.60 | 1.20 |
| Volume average particle diameter (nm) | 210 | 210 | 450 | 210 | 450 | 250 |
| Circularity | > 0.85 | > 0.85 | > 0.85 | > 0.85 | < 0.85 | < 0.85 |

(Preparation of Affinity Particle)

300 Microliters (3 mg in terms of particle solid content) of the dispersion of the particle 1 diluted with ion-exchanged water to a solid content concentration of 1.0 mass% was aliquoted into a 1.5 mL microtube. 90 Microliters of a 5.0% aqueous solution of 1-(3-dimethylaminopropyl)-3-ethylcarbodiimide hydrochloride (Tokyo Chemical Industry Co., Ltd.) and 90 μL of a 5.0% aqueous solution of N-hydroxysulfosuccinimide sodium salt (Tokyo Chemical Industry Co., Ltd.) were added thereto. The mixture was stirred at room temperature for 30 minutes to provide a dispersion of a particle having an activated carboxy group (activated particle dispersion).

After centrifugal washing, 270 μL of phosphate-buffered saline (hereinafter referred to as "PBS") having a pH of 5.5 was added thereto, and the particle having an activated carboxy group was dispersed with an ultrasonic wave.

24 Microliters (0.12 mg in terms of antibody amount) of a 5.0 mg/mL dispersion of a mouse monoclonal anti-ferritin antibody (isoelectric point: 7.1) was added thereto, and the mixture was stirred at room temperature for 3 hours to provide an affinity particle 1 in which the particle 1 was sensitized with the antibody.

Affinity particles 2 to 9, and comparative affinity particles 1 and 2 were prepared by the same experimental operation as that in the preparation of the affinity particle 1 except that the kind of the particle was changed to the particles 2 to 9, and the comparative particles 1 and 2, respectively, in the preparation of the affinity particle 1.

(Preparation of First Reagent)

A first reagent was obtained by dissolving 50 mM HEPES, 0.05 mass% Triton X-100, and 1.0 mass% sodium chloride (Kishida Chemical Co., Ltd.) in ion-exchanged water.

(Preparation of Second Reagent)

After the centrifugal washing of the affinity particle 1, the resultant was re-dispersed in 500 μL of a buffer (HEPES buffer) in which 10 mM HEPES, 0.01 mass% polyoxyethylene nonylphenyl ether (Triton X-100, Kishida Chemical Co., Ltd.), and 10 mass% sucrose (viscosity modifier) was dissolved in ion-exchanged water. After that, the contents were mixed and diluted with the HEPES buffer so that the concentration of the affinity particle 1 became 0.1 mass% to provide a second reagent 1.

Second reagents 2 to 9, and comparative second reagents 1 and 2 were prepared by the same experimental operation as that in the preparation of the second reagent 1 except that the kind of the particle was changed to the affinity particles 2 to 9, and the comparative affinity particles 1 and 2, respectively, in the preparation of the second reagent 1.

(Measurement of Amount of Change in Absorbance)

A mixed solution was prepared by mixing 15 μL of each of specimens prepared to have ferritin concentrations of 0.0 ng/mL (physiological saline) and 100 ng/mL with 60 μL of the first reagent 1, and was kept under the condition of 37°C for 290 seconds. Next, 30 μL of each of the second reagents 1 to 9 and the comparative second reagents 1 and 2 was mixed into the mixed solution, the mixture was stirred, and an absorbance 42 seconds later was measured. Further, the mixed solution was left to stand still at 37°C for 253 seconds, and an absorbance was measured again, and the difference from the absorbance 42 seconds after the stirring was adopted as the amount of change in absorbance ΔABS. The absorbance was measured at a measurement wavelength of 572 nm with a spectrophotometer BIOSPECTROMETER manufactured by Eppendorf Co.

(Calculation of Storage Stability Index)

The amount of change in absorbance at a ferritin concentration of 100 ng/mL was measured, the second reagents 1 to 9, and the comparative second reagents 1 and 2 were left to stand still at 4°C for a certain period, and then the upper half amount of the container was sampled. Those samples were each subjected to measurement of the amount of change in absorbance at a ferritin concentration of 100 ng/mL again under the same conditions, and the amount of change after the lapse of a certain period was calculated. It is expected that as the amount of change becomes smaller, the storage stability is more improved. The storage stability was evaluated as described below based on the value of the storage stability index shown below.

A: The amount of change after the lapse of 14 days was within 10%.
B: The amount of change after the lapse of 7 days was within 10%, but the amount of change after the lapse of 14 days was a value larger than 10%.
C: The amount of change after the lapse of 7 days was a value larger than 10%.

The results are shown in Tables 2-1 and 2-2.

(Calculation of Sensitivity Index)

With regard to the second reagents with a storage stability index of A or B, the sensitivity index was calculated as described below. The amount of change in absorbance when the ferritin concentration was 0.0 ng/mL (physiological saline) was defined as ΔABS(0), and the amount of change in absorbance when the ferritin concentration was 100 ng/mL was defined as ΔABS(100). A value of ΔABS(100)×10,000/ΔABS(0)×10,000 was calculated and adopted as the value of the sensitivity index. It is expected that as the value of the sensitivity index becomes larger, the sensitivity for detecting a target substance becomes higher.

The evaluation was made as described below based on the value of the sensitivity index.

A: The value of the sensitivity index was a value larger than 500.

B: The value of the sensitivity index was a value larger than 100 and 500 or less.

C: The value of the sensitivity index was a value of 100 or less.

The results are shown in Tables 2-1 and 2-2.

Table 2-1

| | Example | | | | | |
|---|---|---|---|---|---|---|
| Affinity particle of second reagent | Affinity particle 1 | Affinity particle 2 | Affinity particle 3 | Affinity particle 4 | Affinity particle 5 | Affinity particle 6 |
| Storage stability | A | A | A | A | B | A |
| Sensitivity | A | A | A | A | A | B |
| Overall evaluation | A | A | A | A | B | B |

Table 2-2

| | Example | | | Comparative Example | |
|---|---|---|---|---|---|
| Affinity particle of second reagent | Affinity particle 7 | Affinity particle 8 | Affinity particle 9 | Comparative affinity particle 1 | Comparative affinity particle 2 |
| Storage stability | A | A | A | C | A |
| Sensitivity | B | B | B | - | C |
| Overall evaluation | B | B | B | C | C |

As described above, the particles 1 to 9 for immunoturbidimetry according to the present disclosure had high sensitivity and excellent storage stability, and both the improvement in sensitivity and the improvement in storage stability were able to be achieved. Meanwhile, the particle of Comparative Example 1 had low storage stability. Further, the particle of Comparative Example 2 had excellent storage stability, but low sensitivity. That is, both the improvement in sensitivity and the improvement in storage stability were not able to be achieved in Comparative Example 1 and Comparative Example 2.

Various embodiments have been described in detail above but it will be understood that the present disclosure is not limited to these embodiments and encompasses all modifications, variants, alternatives and equivalents falling within the scope of the appended claims.

## Claims

**1.** A particle for immunoturbidimetry comprising a first resin and titanium oxide,

wherein the particle for immunoturbidimetry includes a first layer containing the first resin and a second layer containing the titanium oxide,

wherein the second layer is arranged outside of the first layer,
wherein the titanium oxide has a density of 3.40 g/cm$^3$ or less, and
wherein a content of the titanium oxide in the particle for immunoturbidimetry is 10 mass% or more and 80 mass% or less.

2. The particle for immunoturbidimetry according to claim 1, wherein the content of the titanium oxide in the particle for immunoturbidimetry is 21 mass% or more and 60 mass% or less.

3. The particle for immunoturbidimetry according to claim 1 or 2, wherein the particle for immunoturbidimetry has a circularity of 0.85 or more and 1.00 or less.

4. The particle for immunoturbidimetry according to any one of claims 1 to 3, wherein the particle for immunoturbidimetry includes a third layer containing a second resin outside of the second layer containing the titanium oxide.

5. The particle for immunoturbidimetry according to any one of claims 1 to 4, wherein the particle for immunoturbidimetry has a density of 1.20 g/cm$^3$ or more and 1.54 g/cm$^3$ or less.

6. The particle for immunoturbidimetry according to claim 4, wherein the second resin has a structure represented by the following formula (1):

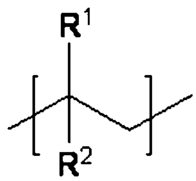

(1)

in the formula (1), $R^1$ represents a hydrogen atom or a methyl group, and $R^2$ represents a structure having a phenyl group that may be substituted or a structure having an ester group.

7. The particle for immunoturbidimetry according to any one of claims 1 to 6, wherein the density of the titanium oxide is 2.50 g/cm$^3$ or more and 3.40 g/cm$^3$ or less.

8. The particle for immunoturbidimetry according to any one of claims 1 to 7, further comprising a ligand on a surface thereof.

9. A reagent comprising the particle for immunoturbidimetry of any one of claims 1 to 8 and an aqueous solution, wherein the particle for immunoturbidimetry is dispersed in the aqueous solution.

10. A test kit comprising the reagent of claim 9 and a container configured to accommodate the reagent.

11. A method of detecting a target substance in a specimen by in vitro diagnosis, the method comprising mixing the reagent of claim 9 and a specimen that may contain the target substance.

12. A method of detecting a target substance in a specimen by in vitro diagnosis, the method comprising:

mixing the reagent of claim 9 with a specimen that may contain the target substance to provide a mixed solution;
irradiating the mixed solution with light; and
detecting at least one of transmitted light and scattered light from the light with which the mixed solution has been irradiated.

Europäisches Patentamt
European Patent Office
Office européen des brevets

# EUROPEAN SEARCH REPORT

Application Number
EP 25 21 8277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 6 683907 B2 (SEKISUI MEDICAL CO LTD; TOTO LTD) 22 April 2020 (2020-04-22) * [0003], [0006], [0009] * ----- | 1-12 | INV. G01N33/543 G01N33/545 G01N33/553 |
| X | JP 2008 241357 A (SANYO CHEMICAL IND LTD) 9 October 2008 (2008-10-09) * [0003]-[0004];[0006], [0011]- [0012], Fig.1;[0016] * ----- | 1-12 | |
| A | JP 2009 073784 A (TOTO LTD) 9 April 2009 (2009-04-09) * the whole document * ----- | 1-12 | |
| A | JP 2008 201797 A (TOTO LTD) 4 September 2008 (2008-09-04) * the whole document * ----- | 1-12 | |

TECHNICAL FIELDS SEARCHED (IPC)

G01N

The present search report has been drawn up for all claims

2

| Place of search | Date of completion of the search | Examiner |
|---|---|---|
| Munich | 20 April 2026 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 21 8277

This annex lists the patent family members relating to the patent documents cited in the above-mentioned European search report. The members are as contained in the European Patent Office EDP file on The European Patent Office is in no way liable for these particulars which are merely given for the purpose of information.

20-04-2026

| Patent document cited in search report | Publication date | Patent family member(s) | Publication date |
|---|---|---|---|
| JP 6683907 B2 | 22-04-2020 | JP 6683907 B2 | 22-04-2020 |
| | | JP 2017122684 A | 13-07-2017 |
| JP 2008241357 A | 09-10-2008 | NONE | |
| JP 2009073784 A | 09-04-2009 | NONE | |
| JP 2008201797 A | 04-09-2008 | JP 4169078 B2 | 22-10-2008 |
| | | JP 2008162995 A | 17-07-2008 |
| | | JP 2008201797 A | 04-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008241357 A